# EUROPÄISCHE PATENTSCHRIFT

(11) **EP 1 636 389 B1**
(45) Veröffentlichungstag und Bekanntmachung des Hinweises auf die Patenterteilung: **22.10.2008**
(21) Anmeldenummer: 04740297.9
(22) Anmeldetag: 25.06.2004
(51) Int. Cl.: C12R 1/19, A61K 35/74, C12N 1/20

(54) **PLASMIDFREIER KLON DES E. COLI-STAMMES DSM 6601**
PLASMID-FREE CLONE OF E. COLI STRAIN DSM 6601
CLONE EXEMPT DE PLASMIDE DE LA SOUCHE D'E. COLI DSM 6601

(30) Priorität: 26.06.2003 DE 10328669
(43) Veröffentlichungstag der Anmeldung: 22.03.2006
(73) Patentinhaber: PHARMA-ZENTRALE GMBH, 58313 Herdecke (DE)
(72) Erfinder: HACKER, Jörg, 97218 Gerbrunn (DE); OELSCHLAEGER, Tobias, 97318 Kitzinger (DE); OSWALD, Sibylle, 97082 Würzburg (DE); SONNENBORN, Ulrich, 44799 Bochum (DE); PROPPERT, Hans, 58095 Hagen (DE)
(74) Vertreter: Schaeffer, Michael
(86) Internationale Anmeldenummer: PCT/EP2004/006886
(87) Internationale Veröffentlichungsnummer: WO 2004/113575

(56) Entgegenhaltungen:
- WO-A-00/78925
- WO-A-98/44134
- WO-A-99/26642
- US-A- 4 686 184
- BLUM ET AL: "Properties of Escherichia coli strains of serotype O6" PLASMID, NEW YORK,NY, US, Bd. 23, Nr. 4, 1. Juli 1995 (1995-07-01), Seiten 234-236, XP002085750 ISSN: 0147-619X
- KRUIS W ET AL: "Einsatz von Probiotika in der Humanmedizin" DIE MEDIZINISCHE WELT, XX, XX, 1996, Seiten 53-57, XP002097855 ISSN: 0025-8512
- CUKROWSKA B ET AL: "Specific proliferative and antibody responses of premature infants to intestinal colonization with nonpathogenic probiotic E. coli strain Nissle 1917." SCANDINAVIAN JOURNAL OF IMMUNOLOGY. FEB 2002, Bd. 55, Nr. 2, Februar 2002 (2002-02), Seiten 204-209, XP002295421 ISSN: 0300-9475
- FIGUEIREDO P P ET AL: "Influence of oral inoculation with plasmid-free human Escherichia coli on the frequency of diarrhea during the first year of life in human newborns." JOURNAL OF PEDIATRIC GASTROENTEROLOGY AND NUTRITION. JUL 2001, Bd. 33, Nr. 1, Juli 2001 (2001-07), Seiten 70-74, XP009036229 ISSN: 0277-2116
- DUVAL-IFLAH Y ET AL: "[Implantation of a strain of "Escherichia coli" in the digestive tract of human new-borns: barrier effect against antibioresistant "E. coli" (author's transl)]" ANNALES DE MICROBIOLOGIE. 1982 MAY-JUN, Bd. 133, Nr. 3, Mai 1982 (1982-05), Seiten 393-408, XP009036261 ISSN: 0300-5410
- TOLKER-NIELSEN T ET AL: "A statistical analysis of the formation of plasmid-free cells in populations of Escherichia coli." JOURNAL OF BACTERIOLOGY. JUL 1994, Bd. 176, Nr. 14, Juli 1994 (1994-07), Seiten 4306-4310, XP009009847 ISSN: 0021-9193

## Beschreibung

Die Erfindung betrifft einen plasmidfreien Klon des E. coli-Stammes DSM 6601, ein Verfahren zu dessen Herstellung sowie die Verwendung der so erhaltenen Bakterien als Klonierungsvehikel.

E. coli, ein in der Natur, insbesondere im menschlichen und tierischen Darm vorkommendes Bakterium, ist bereits seit langem Gegenstand eingehender mikrobiologischer und gentechnologischer Untersuchungen und wird in der Gentechnik insbesondere zur Klonierung und/oder Expression bestimmter Gene bzw. Proteine eingesetzt.

Die meisten Stämme der Gattung Escherichia sind außerhalb des Darmlumens pathogen und führen im allgemeinen zu Infektionen an den befallenen Stellen. Ein apathogener Stamm der Gattung Escherichia coli ist der bei der Deutschen Sammlung für Mikroorganismen hinterlegte Stamm DSM 6601, der in einigen genetischen Merkmalen von allen übrigen E. coli Stämmen abweicht. Es hat sich jedoch gezeigt, dass dieser Stamm nur unter erschwerten Umständen und teilweise überhaupt nicht genetisch manipulierbar ist und sich somit nicht als einfaches Klonierungsmittel einsetzen lässt.

E. coli DSM 6601 enthält natürlicherweise zwei Plasmide, die als pMut1 oder pMut2 bezeichnet werden und eine Größe von 3177 bzw. 5552 kb aufweisen. Diese Plasmide und ihre DNA-Sequenzen sind beispielsweise in der US-6,391,631 beschrieben.

Ausgehend von der Überlegung, dass die Pathogenität bzw. Apathogenität der E. coli-Bakterien offensichtlich teilweise von deren Plasmiden gesteuert wird und der weiteren Überlegung, dass die teilweise auftretende "genetische Resistenz" der E. coli Stämme auch mit dessen kryptischen Plasmiden zusammenhängen könnte, liegt der Erfindung die Aufgabe zugrunde, einen plasmidfreien E. coli Stamm zu entwickeln, der ansonsten hinsichtlich seiner genomischen DNA genetisch völlig identisch mit dem Originalstamm ist.

Die vorstehende Aufgabe wird gelöst durch Bereitstellen eines plasmidfreien Klons des E. coli-Stammes DSM 6601, sowie durch ein Verfahren zur Herstellung eines derartigen Klons.

In den Figuren,

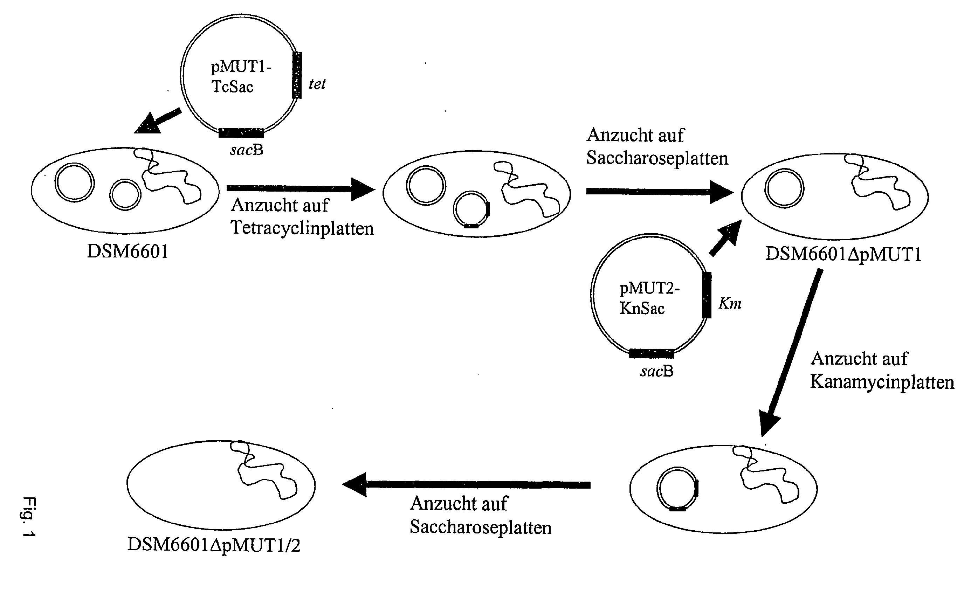

Fig. 1 zeigt in schematischer Form den Verfahrensweg zur Herstellung des plasmidfreien Klons des Stammes DSM 6601;
Fig. 2 zeigt die physikalische Karte des Plasmids pMut1-Tc;
Fig. 3 zeigt physikalische Karte des Plasmides pMut2-Kn.

Bei den langwierigen Untersuchungen, die zur vorliegenden Erfindung führten, hat sich gezeigt, dass plasmidfreie Klone des Stammes DSM 6601 mit normalen gentechnischen Methoden überhaupt nicht oder nur unter großen Schwierigkeiten herstellbar sind, so dass zur Generierung derartiger Klone besondere Wege eingeschlagen werden müssen. Da der Wildtyp des Stammes neben seiner genomischen DNA zwei Plasmide unterschiedlicher Größe aufweist, muß die Eliminierung dieser Plasmide in mehreren, zum Teil parallel laufenden Schritten erfolgen.

Zur Herstellung der erfindungsgemäßen Klone werden gemäß dem erfindungsgemäßen Verfahren die in dem E. coli Stamm DSM 6601 natürlich vorkommenden Plasmide pMut1 und pMut2 in einem ersten Schritt jeweils mit einer Antibiotikaresistenz markiert. Dazu werden die Plasmide gemäß herkömmlicher Verfahren isoliert und das Resistenzgen an einer gewünschten Stelle eingebracht. Gemäß einer bevorzugten Ausführungsform wird das Resistenzgen zusammen mit einer Expressionskasette in das jeweilige Plasmid eingebracht, welche eine Promotor enthält, der konstitutitv oder auch induzierbar sein kann.

Die so erhaltenen Plasmide werden dann gemäß herkömmlicher Verfahren, beispielsweise dem CaCl₂-Verfahren oder Elektroporation, in einen geeigneten Wirt eingebracht, und dort kloniert, wobei das in das jeweilige Plasmid eingebrachte Resistenzgen die Selektion der das Plasmid tragenden Wirtszellen erleichtert. Ein zur Klonierung des ein Resistenzgen tragenden Plasmids geeigneter Wirt sind beispielsweise die E.coli Stämme DH5α oder E.coli HB101.

Die das Resistenzgen tragenden Plasmide werden isoliert und anschließend wird das *sac*B-Gen in die Plasmide eingebracht, um einen weiteren Marker bereitzustellen.

Die so erhaltenen bzw. markierten Plasmide werden dann in den E.coli Stamm DSM 6601 eingebracht.

Nach Transformation werden die E.coli DSM 6601 auf einem Medium gezüchtet, das das Antibiotikum/die Antibiotika enthält, für das/die die Resistenzgene, die in den vorangegangenen Schritten in die Plasmide eingebracht wurde, Resistenz verleihen.

Durch Züchten auf einem derartigen Medium werden nur Klone wachsen, die gegen die im Nährmedium enthaltenen Antibiotika resistent sind. Weiterhin werden die Bakterien, da sie die ursprünglichen Plasmide pMut1 und pMut2 für ihr Wachstum nicht mehr benötigen, überflüssiges genetisches Material verlieren, so dass schlußendlich die Bakterien nur noch die modifizierten Plasmide pMut1 und pMut2 (die das/die Resistenzgene und das sacB Gen enthalten) enthalten.

In einem weiteren Schritt werden die so gezüchteten Bakterien in ein Nährmedium überführt, das das Wachstum von Bakterien hemmt, die das *sacB* enthalten, wobei ein Selektionsdruck ausgeübt wird, der im wesentlichen nur das Wachstum von Bakterien erlaubt, die das *sacB-*Gen verloren haben. Dies kann durch Züchten des Stamms bei 30°C in Gegenwart von 10% Saccharose erfolgen, da sich bei derartigen Bedingungen nur solche Klone replizieren können, die das *sac*B-Gen tragende Plasmid verloren haben.

Als Folge wird ein plasmidfreies Derivat des Stammes DSM 6601 erhalten. Es wurde nun gefunden, dass die erfindungsgemäßen Klone mit Maßgabe des Verlusts der Plasmide keinerlei Veränderung der genomischen DNA erfahren haben und sich überraschenderweise gut als Klonierungsvehikel einsetzen lassen.

Damit können sie im Labor gefahrlos als Wirtszelle für die Klonierung bzw. Expression einer Vielzahl von Genen bzw. Proteinen eingesetzt werden. Versuche mit dem erfindungsgemäßen Stamm DSM 6601 ΔpMut1/2 haben gezeigt, dass er ein besonders guter Akzeptor für Fremd-DNA ist, wenn diese in seine eigenen, in isolierter Form vorliegenden Plasmide integriert wird, d.h. also, die eigenen Plasmide als Klonierungsvektoren für die Fremd-DNA fungieren. Weiterhin können sie, da sie von einem apathogenen Stamm abgeleitet sind zur Behandlung von Störungen des Gastrointestinaltraktes in Tieren und Menschen eingesetzt werden. Dazu können sie, wenn gewünscht, mit Fremdgenen transformiert werden, die die Adhäsion der Bakterien an die Mucosa fördern, wie beispielsweise Adhäsinen, die ggf. Wirtstier-spezifisch die Adhäsion der Bakterien an die Mucosa von beispielsweise Rindern und/oder Schweinen fördern und somit den das Wachstum anderer pathogener Mikroorganismen behindern bzw. verhindern.

Die vorliegende Erfindung wird nun unter Bezug auf die Beispiele weiter erläutert.

### Beispiel 1

### Veränderung von pMut 1

Die beiden natürlich vorkommenden Plasmide pMut1 und pMut2 des Wildtyps DSM 6601 nach dem Plasmid-Midiprep-Protokoll von QIAGEN (QIAGEN Plasmid Purification Handbook 12/2002, Seiten 16-20) isoliert.

Für die Markierung des Plasmids pMut1 wurde die vom Vektor pBR322 abgeleitete Tetracyclinresistenzkassette ausgewählt. Der zugehörige Promotor wurde aus dem Plasmid pASK75 entnommen. Das aus dieser Klonierung resultierende Plasmid wurde mit pKS-tetA^{tetp/o} bezeichnet. Das Insert *tetA*^{tetp/o} (Insertgröße 1,5 kb) wurde mit den Restriktionsenzymen *Xba*I und *Hind*III ausgeschnitten. Das *Xba*I/*Hind*III-Fragment wurde über eine *Nde*I-Restriktionsschnittstelle in das Plasmid pMut1 eingeführt.

Dazu wurde nach Restriktionsverdau der Plasmide mit den entsprechenden Enzymen der Restriktionsansatz über eine Säule (Quiagen, PCR Purification Kit) gereinigt und einer Klenow-Behandlung unterzogen, um "blunt ends" zu bilden. Um eine Religation des Plasmids pMut1 zu verhindern, wurde eine Dephosphorylierung des mit dem Restriktionsenzym *Nde*I linearisierten und mit Klenow-Enzym behandelten Plasmids durchgeführt. Anschließend wurde der Vektor pMut1 und das Insert *tetA*^{tetp/o} ligiert und damit der *E. coli* K-12 Stamm DH5α transformiert.

Zur Herstellung kompetenter Zellen wurden 150 ml LB-Medium (Lurea-Bertani-Medium) mit 1,5 ml einer ÜN-Kultur beimpft und bei 37°C geschüttelt bis zu einer OD₆₀₀=0,5. Danach wurde die Bakterienkultur 20 Minuten auf Eis inkubiert und 10 Minuten mit 4000 Upm bei 4°C zentrifugiert. Das Bakterienpellet wurde 3 x in sterilem, eiskaltem, 10%-igem Glycerin gewaschen, zuerst mit 100%, dann mit 50% und zuletzt mit 10% des Ausgangsvolumens. Zum Schluß wurde das gewaschene Pellet in 300 µl 10% Glycerin resuspendiert, aliquotiert (40 µl) und bei -80°C gelagert. Zur Transformation von Bakterienzellen wurden 1-2 µl Plasmid-DNA (1-100 ng) mit 40 µl auf Eis aufgetauten kompetenten Zellen gemischt und 5 Minuten auf Eis inkubiert. Danach wird dieser Ansatz luftblasenfrei zwischen die beiden Elektroden einer sterilen und vorgekühlten 2 mm Elektroporationsküvette pipettiert. Die Küvette wird gut abgetrocknet und in den Elektrodenhalter eingesetzt. Nach Durchführung des Elektroimpulses bei 2,5 kV, 200 Ω und 25 µF wurde die Bakteriensuspension mit 1 ml LB-Medium aus der Küvette gespült und bei 37°C für 1-2 Std. im Schüttler inkubiert. Anschließend wurden die Bakterien abzentrifugiert und der Überstand bis auf 100 µl abgezogen. Das Sediment wurde in den verbliebenen 100 µl resuspendiert, auf einer Tc-haltigen Selektionsplatte ausplattiert und ÜN bei 37°C inkubiert.

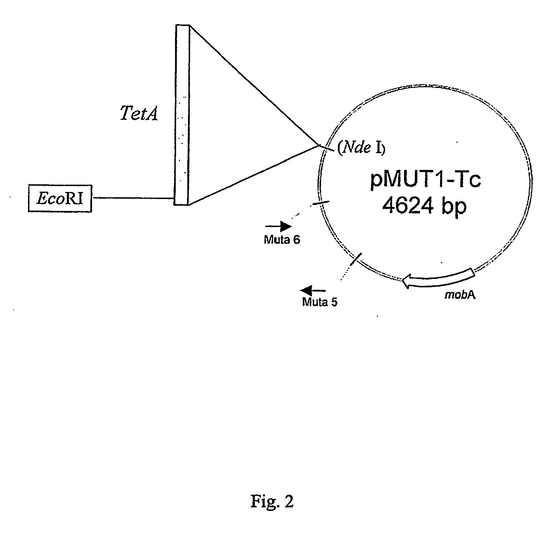

Der Erfolg der Klonierung wurde durch Minipräparation des jeweiligen Plasmids einzelner Bakterienklone überprüft. Das mit der Tetracyclinkassette markierte Plasmid pMut1 wurde als pMut1-Tc bezeichnet. In Fig. 2 ist die physikalische Karte des Plasmids pMut1-Tc dargestellt. Angegeben ist die Insertionsstelle (ursprünglich eine singuläre *Nde*I-Stelle) für das DNA-Fragment, welches Tetracyclinresistenz vermittelt. Dieses DNA-Fragment enthält auch die für Klonierungen geeignete singuläre EcoRI-Sequenz. Des weiteren sind die Bindungsstellen für die Primer Muta 5 und Muta 6 angegeben, die zum spezifischen Nachweis dieses Plasmids mittels PCR geeignet sind.

### Beispiel 2

### Veränderung von pMut2

Für die Markierung des Plasmides pMut2 wurde eine Kanamycinresistenzkassette ausgewählt, die von dem Vektor pACYC177 abgeleitet wurde. Dazu wurde aus diesem die Resistenzkassette (Größe 1,34 kb) mit dem Restriktionsenzym *Stu*I ausgeschnitten und über eine *Bgl*II-Restriktionsschnittstelle in das Plasmid pMut2 eingeführt. Nach Restriktionsverdau der Plasmide mit den entsprechenden Enzymen wurde der Restriktionsansatz über eine Säule (Quiagen, PCR Fraktion Kit) gereinigt und das Plasmid pMut2 einer Klenow-Behandlung unterzogen, um "blunt ends" für die Klonierung zu bilden. Um eine Religation des Plasmides pMut2 zu verhindern, wurde eine Dephosphorylierung des mit dem Restriktionsenzym *Bgl*II linearisierten und mit Klenow-Enzym behandelten Plasmides durchgeführt. Anschließend wurde der Vektor pMut2 und die Kanamycinkassette ligiert und damit der *E. coli* K-12 Stamm DH5α, wie in Beispiel 1 beschrieben, transformiert. Die Transformation wurde auf Kn-haltigen LB-Agarplatten ausplattiert. Der Erfolg der Klonierung wurde durch Minipräparation von Plasmid-DNA einzelner Bakterienklone überprüft. Das mit der Kanamycinkassette markierte Plasmid pMut2 wurde als pMut2-Kn bezeichnet.

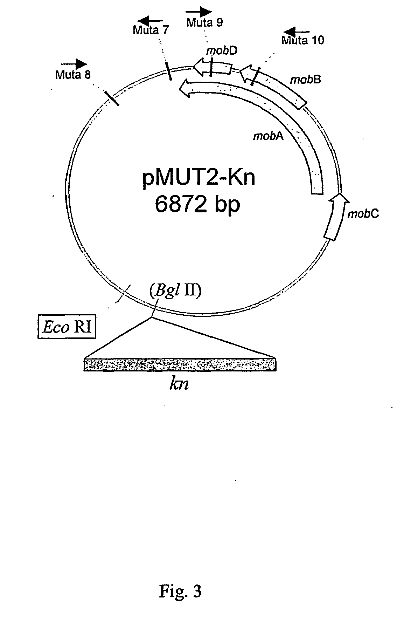

In Fig. 3 ist die physikalische Karte des Plasmides pMut2-Kn dargestellt. Die Insertionsstelle (ursprünglich eine *Bgl*II-Stelle) für die Kanamycinresistenzkassette sowie die singuläre und als Klonierungsstelle geeignete *Eco*RI-Sequenz sind eingezeichnet. Mit Muta 7 bis Muta 10 sind jene Bereiche gekennzeichnet, die komplementär zu den Sequenzen der Primer sind, die für die spezifischen Nachweis dieses Plasmids mittels PCR geeignet sind.

### Beispiel 3

### Einbringen des sacB-Gens

Um das *sac*B-Gen (kodiert für eine Levansaccharase) in die mit Resistenzkassetten markierten Plasmide pMut1-Tc und pMut2-Kn einzubringen, wurde in beiden Plasmiden die jeweils singuläre *Eco*RI-Restriktionsschnittstelle ausgewählt. Das *sac*B-Gen (Größe 2,6 kb) wurde aus dem Plasmid pCVD442 mit dem Restriktionsenzym *Pst*I isoliert. Zur Vorbereitung der Vektoren pMut1-Tc und pMut2-Kn wurden diese Plasmide mit *Eco*RI linearisiert und anschließend zur Bildung von "blunt ends" einer Klenow-Behandlung unterzogen sowie dephosphoryliert. Das Insert *sac*B wurde nach dem Restriktionsverdau mit *Pst*I gereinigt und mit Klenow-Enzym behandelt. Die linearisierten Vektoren und das Insert wurden ligiert und damit der *E. coli* K-12 Stamm DH5α, der wie in Beispiel 1 beschrieben kompetent gemacht wurde, transformiert. Der Erfolg der Klonierung wurde durch Minipräparation von Plasmid-DNA einzelner Bakterienklone und anschließende Restriktionsanalyse überprüft. Die mit dem *sac*B-Gen markierten Plasmide pMut1-Tc und pMut2-Kn wurden als pMut1-TcSac und pMut2-KnSac bezeichnet.

### Beispiel 4

### Herstellung eines plasmidfreien Klones des E. coli-Stammes DSM 6601

Zunächst wurde das Plasmid pMut1-TcSac durch Elektroporation in diesen Stamm, wie in Beispiel 1 beschrieben, transformiert. Nach der Elektroporation zur Überführung des Plasmides pMut1-TcSac in den Stamm DSM 6601 wurde der Ansatz auf Tc-haltigen (50 µg/ml) LB-Platten ausplattiert. Die erhaltenen Tetracyclin-resistenten Bakterienklone wurden auf den Besitz des Plasmids pMut1-TcSac und den damit verbundenen Verlust des natürlich vorkommenden Plasmides pMut1 überprüft. Der Verlust des Plasmids pMut1 wurde nach Präparation der Plasmid DNA und Restriktionsverdau derselben mit dem Enzym *Eco*RI nach elektrophoretischer Auftrennung der linearisierten Plasmide durch das Fehlen der pMut1 repräsentierenden DNA-Bande nachgewiesen.

Anschließend wurde einer dieser Klone über Nacht in LB-Medium mit 10% Saccharose bei 30°C angezogen und dann auf LB-Platten mit 10% Saccharose ausplattiert (LB-Medium besteht aus 10 g Pepton aus Casein, 5 g Hefeextrakt und 5 g Natriumchlorid auf 1 1 destilliertem Wasser). Saccharose-haltiges LB-Medium wurde hergestellt, indem dem autoklavierten Medium nach dem Abkühlen auf 45°C von einer steril filtrierten 50 prozentigen (Gew./Vol.) Saccharosestammlösung ein Anteil bis zu einer Endkonzentration von 10% zugegeben wird.) Die Platten wurden bei 30°C inkubiert. Unter diesen Bedingungen können nur jene Klone replizieren, die *sac*B nicht mehr exprimieren, d.h. diese das *sac*B-Gen tragende Plasmid verloren haben. Der daraus resultierende Stamm DSM 6601ΔpMut1 wurde auf den Verlust des Plasmides pMut1-TcSac überprüft.

Danach wurde durch Elektroporation das Plasmid pMut2-KnSac in den Stamm DSM 6601ΔpMut1 eingebracht.

Nach der Elektroporation zur Überführung des Plasmides pMut2-KnSac in den Stamm DSM 6601ΔpMut1 wurde der Ansatz auf Kn-haltigen (50 µg/ml) LB-Platten ausplattiert. Die erhaltenen Kanamycin-resistenten Bakterienklone wurden auf den Besitz des Plasmids pMut2-KnSac und den damit verbundenen Verlust des natürlich vorkommenden Plasmides pMut2 überprüft. Anschließend wurde einer dieser Klone über Nacht in LB-Medium mit 10% Saccharose bei 30°C angezogen und dann auf LB-Platten mit 10% Saccharose ausplattiert. Die Platten wurden bei 30°C inkubiert. Der daraus resultierende Stamm DSM 6601 ΔpMut1/2 wurde auf den Verlust des Plasmides pMut2-KnSac überprüft.

Weiterhin wurde von dem plasmidfreien Stamm DSM 6601 ΔpMut1/2 eine Pulsfeldgelelektrophorese durchgeführt, um evtl. chromosomale Veränderungen des Stammes auszuschließen. Es wurde festgestellt, dass keine Änderungen nachweisbar waren und weitere Untersuchungen haben ergeben, dass der plasmidfreie Klon keinerlei morphologische, biochemische oder fermentative Änderungen zeigt.

## Patentansprüche

1. Plasmidfreier Klon des Escherichia coli-Stammes DSM 6601.

2. Verfahren zur Herstellung eines plasmidfreien Klons nach Anspruch 1, **gekennzeichnet durch** die folgenden Schritte:
a) Einbringen eines Resistenzgens in die Plasmide pMut1 und pMut2,
b) Einbringen des *sac*B-Gens in die in Schritt a) erhaltenen Plasmide,
c) Einbringen der in Schritt b) erhaltenen Plasmide in den E.coli-Stamm DSM 6601 und Züchten des Stamms unter Bedingungen, bei denen die natürlich vorkommenden Plasmide pMut1 und pMut2 **durch** die in Schritt b) erhaltenen Plasmide verdrängt werden; und
d) Züchten der in Schritt c) erhaltenen Klone unter Bedingungen, die im wesentlichen nur ein Wachstum von Bakterien erlaubt, denen das *sac*B-Gen fehlt.

3. Verfahren nach Anspruch 2, **dadurch gekennzeichnet, dass** die Resistenzgene in einer Expressionskasette vorliegen.

4. Verfahren nach Anspruch 2 oder 3, **dadurch gekennzeichnet, dass** die Resistenzgene ausgewählt sind unter Tetracyclinresistenz oder Kanamycinresistenz.

5. Verfahren nach einem der Ansprüche 2 bis 4, **dadurch gekennzeichnet, dass** das Plasmid pMut1 mit einer Tetracyclinresistenzkassette und dem *sac*B-Gen markiert ist und dass das ursprüngliche Plasmid pMut2 mit einer Kanamycinresistenzkassette und dem *sac*B-Gen markiert ist.

6. Verfahren nach einem der Ansprüche 2 bis 5, wobei die mit dem Plasmid pMut1, das mit einer Tetracyclinresistenzkassette und dem *sac*B-Gen markiert ist, transformierten Bakterien auf Tetracyclin enthaltenden Platten und anschließend auf Saccharose enthaltenden Platten gezüchtet werden, und dass nach Eliminierung des Plasmids pMut1 im ersten Schritt die Eliminierung des Plasmids pMut2 durch Anzucht auf Kanamycinplatten und Weiterkultivierung auf Saccharoseplatten erfolgt.

7. Verwendung der plasmidfreien Klone nach Anspruch 1 als Klonierungsstämme.

8. Verwendung der plasmidfreien Klone nach Anspruch 1 zur Herstellung eines Mittels zur Behandlung von gastrointestinalen Störungen in Tieren.

## Claims

1. Plasmid-free clone of Escherichia coli strain DSM 6601.

2. Process for the preparation of a plasmid-free clone according to claim 1,
**characterized by** the following steps:
a) introduction of a resistance gene into the plasmids pMut1 and pMut2,
b) introduction of the *sac*B gene into the plasmids obtained in step a),
c) introduction of the plasmids obtained in step b) into the E. coli strain DSM 6601 and culture of the strain under conditions under which the naturally occurring plasmids pMut1 and pMut2 are displaced by the plasmids obtained in step b); and
d) culture of the clones obtained in step c) under conditions which essentially allow only a growth of bacteria which lack the *sac*B gene.

3. Process according to claim 2, **characterized in that** the resistance genes are present in an expression cassette.

4. Process according to claim 2 or 3, **characterized in that** the resistance genes are chosen from tetracycline resistance or kanamycin resistance.

5. Process according to one of claims 2 to 4, **characterized in that** the plasmid pMut1 is labelled with a tetracycline resistance cassette and the *sac*B gene, and **in that** the original plasmid pMut2 is labelled with a kanamycin resistance cassette and the *sac*B gene.

6. Process according to one of claims 2 to 5, wherein the bacteria transformed with the plasmid pMut1, which is labelled with a tetracycline resistance cassette and the *sac*B gene, are cultured on tetracycline-containing plates and then on sucrose-containing plates, and in that after elimination of the plasmid pMut1 in the first step, elimination of the plasmid pMut2 takes place by culture on kanamycin plates and further culturing on sucrose plates.

7. Use of the plasmid-free clones according to claim 1 as cloning strains.

8. Use of the plasmid-free clones according to claim 1 for the preparation of an agent for treatment of gastrointestinal disorders in animals.

## Revendications

1. Clone de la souche *Escherichia coli* DSM-6601, sans plasmide.

2. Procédé de préparation d'un clone sans plasmide, conforme à la revendication 1, **caractérisé en ce qu'**il comporte les étapes suivantes :
a) introduire un gène de résistance dans des plasmides pMUT1 et pMUT2 ;
b) introduire le gène *sac*B dans les plasmides obtenus dans l'étape (a) ;
c) introduire les plasmides obtenus dans l'étape (b) chez la souche *Escherichia coli* DSM-6601 et cultiver cette souche dans des conditions dans lesquelles les plasmides naturels pMUT1 et pMUT2 sont supplantés par les plasmides obtenus dans l'étape (b) ;
d) cultiver les clones obtenus dans l'étape (c), dans des conditions qui font que, pratiquement, seules peuvent croître les bactéries dépourvues du gène *sac*B.

3. Procédé conforme à la revendication 2, **caractérisé en ce que** le gène de résistance se trouve au sein d'une cassette d'expression.

4. Procédé conforme à la revendication 2 ou 3, **caractérisé en ce que** le gène de résistance est choisi parmi un gène de résistance à la tétracycline et un gène de résistance à la kanamycine.

5. Procédé conforme à l'une des revendications 2 à 4, **caractérisé en ce que** le plasmide pMUT1 est marqué avec une cassette de résistance à la tétracycline et avec le gène *sac*B, et **en ce que** le plasmide originel pMUT2 est marqué avec une cassette de résistance à la kanamycine et avec le gène *sac*B.

6. Procédé conforme à l'une des revendications 2 à 5, **caractérisé en ce que** les bactéries transformées avec le plasmide pMUT1 marqué avec une cassette de résistance à la tétracycline et avec le gène *sac*B sont cultivées sur des plaques contenant de la tétracycline, puis sur des plaques contenant du saccharose, et **en ce qu'**après avoir éliminé le plasmide pMUT1 dans cette première étape, on élimine le plasmide pMUT2 en opérant une culture sur des plaques contenant de la kanamycine, puis une culture sur des plaques contenant du saccharose.

7. Emploi d'un clone sans plasmide, conforme à la revendication 1, en tant que souche de clonage.

8. Emploi d'un clone sans plasmide, conforme à la revendication 1, en vue de la préparation d'un agent conçu pour le traitement de troubles gastro-intestinaux chez des animaux.
